# EUROPEAN PATENT SPECIFICATION

(11) **EP 0 437 025 B1**
(45) Date of publication and mention of the grant of the patent: **06.09.1995**
(21) Application number: 90312053.3
(22) Date of filing: 02.11.1990
(51) Int. Cl.: A61K 31/415

(54) **Compositions containing aldose reductase inhibitors for the treatment of ulcers**
Aldosereduktasehemmer enthaltende Zubereitungen zur Behandlung von Geschwüren
Compositions contenant des inhibiteurs de l'aldose réductase pour le traitement des ulcères

(30) Priority: 12.01.1990 JP 5773/90
(43) Date of publication of application: 17.07.1991
(73) Proprietor: SANWA KAGAKU KENKYUSHO CO., LTD., Higashi-ku Nagoya-shi Aichi-ken (JP)
(72) Inventor: Sawai, Kiichi, c/o Sanwa Kagaku Kenkyusho Co. Ltd., Higashi-ku, Nagoya-shi, Aichi-ken (JP); Kuroni, Masayasu, c/o Sanwa Kagaku Kenkyusho Co., Higashi-ku, Nagoya-shi, Aichi-ken (JP); Nakano, Kazumasa, c/o Sanwa Kagaku Kenkyusho Co., Higashi-ku, Nagoya-shi, Aichi-ken (JP); Sato, Makoto, c/o Sanwa Kagaku Kenkyusho Co., Ltd., Higashi-ku, Nagoya-shi, Aichi-ken (JP); Kuboyama, Noboru, c/o Sanwa Kagaku Kenkyusho Co., Higashi-ku, Nagoya-shi, Aichi-ken (JP); Ito, Takashi, c/o Sanwa Kagaku Kenkyusho Co., Ltd., Higashi-ku, Nagoya-shi, Aichi-ken (JP); Kondo, Yoshiya, c/o Sanwa Kagaku Kenkyusho Co., Higashi-ku, Nagoya-shi, Aichi-ken (JP)
(74) Representative: Dixon, Donald Cossar

(56) References cited:
- EP-A- 0 264 586
- EP-A- 0 384 416
- GB-A- 2 054 373
- US-A- 4 600 717
- US-A- 4 717 725
- CHEMICAL ABSTRACTS, vol. 110, no. 11, 13th March 1989, page 78, abstract no.88588w, Columbus, Ohio, US; N. IBARAKI et al.: "Wound healing of corneal endothelia of galactosemic rats" & ATARASHII GANKA 1988,5(11), 1643-50
- CHEMICAL ABSTRACTS, vol. 102, no. 15, 15th April 1985, page 63, abstract no.125565u, Columbus, Ohio, US; H. ONO et al.: "Effect of aldose reductase inhibitor M79175 on reepithelialization of denuded corneas in streptozotocin-diabetic rats" & ATARASHII GANKA 1984, 1(3), 421-3
- JOURNAL OF OCULAR PHARMACOLOGY, vol. 4, no. 3, 1988, pages 195-201, Mary Ann Liebert, Inc.; T. AWATA et al.: "Effect of an aldose reductase inhibitor, CT-112, on healing of the corneal epithelium in galactose-fed rats"
- "THE MERCK INDEX - AN ENCYCLOPEDIA OF CHEMICALS, DRUGS, AND BIOLOGICALS", 11th edition, 1989, editor S. Budavari et al., Merck & Co.,Inc., Rahway, N.J., US;

## Description

This invention relates to a therapeutic composition for the treatment of ulcers and containing an aldose reductase as a primary component. The compositions may be administered externally for the treatment gastro-intestinal tract ulcers.

In recent years, it has been found that one of the causes of cataract, retinitis and various nervous disorders induced by diabetes is an intracellular accumulation of sorbitol formed by way of the polyol pathway, and attention has been paid to various aldose reductase inhibitory substances, because enzymatic inhibition of the exchange between aldose and polyol reduces the production or accumulation of sorbitol.

The applicant has already filed patent applications to patent per se the compounds used in this invention (see Japanese Patent Kokai Publication No. 61(1986)-200991 and US Patent No. 4,861,792).

Hitherto, many aldose reductase inhibitors have been studied for treating diabetic complications (see US Patent No. 4,900,739). Further, both US-A-4717725 and US-A-4600717 disclose the treatment of wounds with aldose reductase inhibitors exemplified by the treatment of ocular wounds in diabetic patients. However, it has not previously been known that aldose reductase inhibition systems take part in the promotion of tissue metabolism, and that they also have an effect upon inhibiting ulceration.

According to the present invention a use is provided, in the manufacture of a medicament for the treatment of ulcers of the stomach and gastrointestinal tract by oral administration, of an antiulcer effective amount of a compound selected from d-6-fluoro-2,3-dihydro-2',5'-dioxo-spiro[4H-1-benzopyran-4,4'-imidazolidine]-2-carboxyamide, d-2-chloromethyl-6-fluoro-2,3-dihydro-spiro[4H-1-benzopyran-4,4'-imidazolidine]-2',5'-dione and d-2-bromomethyl-6-fluoro-2,3-dihydro-spiro[4H-1-benzo-pyran-4,4'-imidazolidine]-2',5'-dione. If desired, the hydantoin derivatives of the present invention may be provided in the form of a composition to which stabilizers, absorption accelerators have been added. Examples of the carriers that may be used are carboxylmethylcellulose, polyvinyl pyrrolidone and cyclodextrin.

The therapeutic compositions according to this invention activate tissue metabolism and so are efficacious against all aspects of exhaustion tissue necroses particularly ulcers of the stomach and gastro-intestinal tract.

The present invention will now be illustrated more specifically with reference to pharmacological Tests and non-limiting Examples.

### EXAMPLES

### Preparation Examples

Three hydantoin derivatives, i.e., d-6-fluoro-2,3-dihydro-2',5'-dioxo-spiro [4H-1-benzopyran-4,4'-imidazolidine]-2-carboxyamide (hereinafter called Compound A), d-2-chloromethyl-6-fluoro-2,3-dihydro-spiro [4H-1-benzopyran-4,4'-imidazolidine]-2',5'-dione (hereinafter called Compound B) and d-2-bromomethyl-6-fluoro-2,3-dihydro-spiro [4H-1-benzopyran-4,4'-imidazolidine]-2',5'-dione (hereinafter called Compound C) are used to obtained the following forms of preparation (a) suppository and (b) tablet:

### (a) Suppository

| | |
|---|---|
| Compound A | 100 mg |
| Cacao butter | 1600 mg |
| | 1700 mg per suppository |

Compound A is dispersed in a cacao butter (higher fatty acid glyceride) melt that is an oil and fat base, and then formed in conventional manner to obtain suppositories.

### (b) Tablet

| | |
|---|---|
| Compound A | 50 g |
| Sodium citrate | 25 g |
| Arginine | 10 g |
| Polyvinyl pyrrolidone | 10 g |
| Magnesium stearate | 5 g |

In conventional manner the above-mentioned components are tableted to prepare 1000 tablets for oral administration, each containing 50 mg of the active component.

### Pharmacological Test Example 1

### Effect on Inhibiting Ulcer Induced by Water Immersion Stress

After 24-hour fasting, S.D. masculine rats weighing 250-270 g were immersed to their breasts in a water tank maintained at 23 1 to load a water immersion stress on them. Seven hours later, the stomachs were evulsed and filled with 10 ml of a 2% formalin solution according to the method described in "Jap. J. Pharmac.", 18, pp. 9-18 (1968) for temporal fixation. The stomachs were incised to find the sum of lengths of ulcerated regions on the stomach bodies - an ulcer factor. A solution of 20 mg/kg of the instant compound dissolved in physiological saline was orally administered to the animals 10 minutes before stress loading.

As reported in Table 1, the instant compound showed an inhibitory action upon the ulceration of the stomach bodies.

**Table 1**

| | | Number of animals | Ulcer Factor |
|---|---|---|---|
| Control Group | | 10 | 14.8 2.0 |
| Compound | A | 10 | 11.5 1.5 |
| | B | 10 | 10.6 1.2 |
| | C | 10 | 12.2 2.6 |
| Sorbinil | | 10 | 12.6 1.8 |

## Claims

1. The use of an antiulcer effective amount of a compound selected from d-6-fluoro-2,3-dihydro-2',5'-dioxo-spiro[4H-1-benzopyran-4,4'-imidazolidine]-2-carboxyamide, d-2-chloromethyl-6-fluoro-2,3-dihydro-spiro[4H-1-benzopyran-4,4'-imidazolidine]-2',5'-dione and d-2-bromomethyl-6-fluoro-2,3-dihydro-spiro[4H-1-benzo-pyran-4,4'-imidazolidine]-2',5'-dione in the manufacture of a medicament for the treatment of ulcers of the stomach and gastro-intestinal tract by oral administration.

## Patentansprüche

1. Verwendung einer antigeschwürwirksamen Menge einer Verbindung, ausgewählt aus d-6-Fluor-2,3-dihydro-2',5'-dioxo-spiro[4H-1-benzopyran-4,4'-imidazolidin]-2-carboxyamid, d-2-Chlormethyl-6-fluor-2,3-dihydro-spiro[4H-1-benzopyran-4,4'-imidazolidin]-2',5'-dion und d-2-Brommethyl-6-fluor-2,3-dihydro-spiro[4H-1-benzopyran-4,4'-imidazolidin]-2',5'-dion, in der Herstellung eines Medikaments zur Behandlung von Geschwüren des Magens und des Gastrointestinaltraktes durch orale Verabreichung.

## Revendications

1. Utilisation d'une quantité, efficace pour un effet anti-ulcère, d'un composé choisi parmi le d-6-fluoro-2,3-dihydro-2',5'-dioxo-spiro[4H-1-benzopyran-4,4'-imidazolidine]-2-carboxyamide, la d-2-chlorométhyl-6-fluoro-2,3-dihydro-spiro[4H-1-benzopyran-4,4'-imidazoline]-2',5'-dione, et la d-2-bromométhyl-6-fluoro-2,3-dihydro-spiro[4H-1-benzo-pyran-4,4'-imidazolidine]-2',5'-dione, dans la fabrication d'un médicament destiné à être administré par voie orale pour traiter des ulcères de l'estomac et du tube digestif (gastro-intestinal).
